# EUROPÄISCHE PATENTSCHRIFT

(11) **EP 2 097 118 B1**
(45) Veröffentlichungstag und Bekanntmachung des Hinweises auf die Patenterteilung: **18.09.2013**
(21) Anmeldenummer: 07845634.0
(22) Anmeldetag: 17.12.2007
(51) Int. Cl.: A61F 2/30, A61C 8/00, A61L 27/06, A61L 27/30, A61L 27/54

(54) **BIOAKTIVE IMPLANTATBESCHICHTUNG**
BIOACTIVE IMPLANT COATING
REVETEMENT D'IMPLANT BIOACTIF

(30) Priorität: 21.12.2006 CH 21012006
(43) Veröffentlichungstag der Anmeldung: 09.09.2009
(73) Patentinhaber: THOMMEN MEDICAL AG, 4437 Waldenburg (CH)
(72) Erfinder: BAYER, Ulrich, 76307 Karlsbad-Spielberg (DE); SCHMIDT, Jürgen, 07745 Jena (DE); HENNING, Angelika, 07751 Zöllnitz (DE); KAUTZ, Armin Rex, 07745 Jena (DE); WEISSER, Jürgen, 99092 Erfurt (DE); SCHNABELRAUCH, Matthias, 07749 Jena (DE); SCHLOTTIG, Falko, 4414 Füllinsdorf (CH)
(74) Vertreter: Bremi, Tobias Hans
(86) Internationale Anmeldenummer: PCT/CH2007/000637
(87) Internationale Veröffentlichungsnummer: WO 2008/074175

(56) Entgegenhaltungen:
- EP-A- 0 232 791
- WO-A-02/078759
- WO-A-2006/104644
- DE-A1-102004 021 739
- US-A- 5 478 237
- US-A- 5 811 194
- MARIE P J ET AL: "MECHANISMS OF ACTION AND THERAPEUTIC POTENTIAL OF STRONTIUM IN BONE" CALCIFIED TISSUE INTERNATIONAL, NEW YORK, NY, US, Bd. 69, Nr. 3, 1. Januar 2001 (2001-01-01), Seiten 121-129, XP008040696 ISSN: 0171-967X in der Anmeldung erwähnt

## Beschreibung

### TECHNISCHES GEBIET

Die vorliegende Erfindung betrifft eine die Osteointegration verbessernde Beschichtung für metallische Implantate zum wenigstens teilweisem Einsetzen in den Knochen. Weiterhin betrifft die vorliegende Erfindung ein Verfahren zur Herstellung einer solchen Beschichtung und zu Verwendungen der Implantate.

### STAND DER TECHNIK

Verletzte oder beschädigte Teile des Hartgewebes des menschlichen Körpers werden am Besten wiederhergestellt oder mechanisch verstärkt, indem körpereigenes Hartgewebe verwendet wird. Dies ist aus verschiedenen Gründen nicht immer möglich, und daher kommt in vielen Fällen synthetisches Material als temporäres (bioabbaubares respektive postoperativ entfernbares) oder permanentes Ersatzmaterial zum Einsatz.

Implantate, welche hauptsächlich im Hartgewebe verankert werden, dienen dem temporären oder permanenten Ersatz oder dem Support von unfall-, abnützungs-, mangelerscheinungs- oder krankheitsgeschädigten oder sonst degenerierten Teilen des menschlichen Körpers. Als Implantat wird normalerweise ein künstliches, chemisch stabiles Material bezeichnet, welches als plastischer Ersatz oder zur mechanischen Verstärkung in den Körper eingebracht wird (vgl. z.B. Roche Lexikon Medizin, Urban & Fischer, (Hrsg.); 5. Aufl. 2003). Die Hilfs- und Ersatzfunktion im Körper wird auf Basis der mechanischen Eigenschaften und des Implantatdesigns übernommen. So sind beispielsweise Hüft- und Kniegelenksprothesen, Wirbelsäulenimplantate und Materialien zur Osteosynthese (Versorgung von Knochenfrakturen) seit vielen Jahren im erfolgreichen klinischen Einsatz. Auf Grund der stabilisierenden, lastaufnehmenden und/oder -übertragenden Funktion, die solche Implantate übernehmen sollen, werden als Werkstoffe zu ihrer Herstellung sehr oft Metalle oder metallische Legierungen eingesetzt. Typische Metalle oder metallische Legierungen, die für Implantate im Hartgewebebereich eingesetzt werden, sind Titan, Titanlegierungen, Edelstahl oder Kobalt-Chrom-Legierungen. Für die Implantatverankerung und die Implantatverträglichkeit an der Grenzfläche Implantatoberfläche / angrenzendes Gewebe hat die Implantatoberfläche eine grosse Bedeutung. Durch eine Veränderung der Implantatoberfläche kann der Heilungsprozess beschleunigt werden.

Zur Oberflächenbehandlung und Oberflächenstrukturierung werden verschiedenste Methoden verwendet, siehe z. B. Titanium in Medicine, Material Science, Surface Science, Engineering, Biological Responses and Medical Applications Series: Engineering Materials, (Brunette, D.M.; Tengvall, P.; Textor, M.; Thomsen, P.(Eds.)); und die darin genannten Referenzen.

Gut etabliert sind Calciumphosphathaltige Oberflächenschichten, die beispielsweise durch thermische Spritzverfahren, Sol-Gel-Verfahren oder elektrochemische Verfahren auf Implantatoberflächen erzeugt werden können. Calciumphosphat-Beschichtungen weisen auf Grund ihrer chemischen Ähnlichkeit mit der mineralischen Phase des Knochens eine sehr gute Biokompatibilität auf. In Abhängigkeit von der Löslichkeit der Calciumphosphat-Beschichtung können Calcium- und Phosphat-Ionen in den Knochen eingebaut werden, wodurch ein stabilerer Verbund zwischen Implantat und umliegendem Knochengewebe entsteht. Darüber hinaus kann die Calciumphosphat-Beschichtung so ausgebildet werden, dass eine offen-poröse Struktur entsteht, in die Knochenzellen einwachsen und sich verankern können. Diese als Osteokonduktivität bezeichnete Eigenschaft der Beschichtung fördert eine schnelle Stabilisierung des Implantats im umliegenden Gewebe.

Ein wesentlicher Nachteil von Calciumphosphat-Beschichtungen ist allerdings darin zu sehen, dass diese Beschichtungen den Heilungsprozess des Knochengewebes nicht aktiv stimulieren, also nicht osteogen wirken. Es wurden daher in jüngster Zeit grosse Anstrengungen unternommen, Implantatoberflächen mit Substanzen zu beschichten, die eine osteogene oder osteoinduktive Wirkung entfalten können. Als Substanzen mit osteoinduktiver Wirkung gelten beispielsweise Wachstumsfaktoren, wie die sogenannten Bone Morphogenetic Proteins (BMP). Auch niedermolekulare Substanzen sind in der Lage, die Prozesse der Knochenbildung und des Knochenabbaus zu beeinflussen. Als solche osteogene Substanzen sind aus der Literatur beispielsweise Bisphosphonate, Statine oder wasserlösliche Strontiumsalze (z. B. Strontiumranelat) bekannt. Gegenwärtig werden diese Substanzen in der Osteoporose-Behandlung bereits eingesetzt (siehe z. B. Biskoping, D. M.: Expert Opin. Invest. Drugs 12 (2003), 611-621). Solche niedermolekularen Verbindungen sind zur Erzielung osteogener Eigenschaften von Implantatoberflächen von besonderem Interesse, da sie im Vergleich zu Proteinen wie BMP in der Regel weniger empfindlich gegenüber herkömmlichen Sterilisationsverfahren für Implantate sind. Unter den niedermolekularen Substanzen mit potentiell osteogener Wirkung sind wasserlösliche Strontiumsalze von besonderem Interesse, da bekannt ist, das diese Verbindungen sowohl knochenbildende Zellen (Osteoblasten) aktivieren als auch knochenabbauende Zellen (Osteoklasten) hemmen (Marie, P. J., Ammann, P., Boivin, G., Rey, C.: Calcif. Tissue Intern. 69 (2001),121-129). Wasserlösliche Strontiumsalze wie Strontiumranelat sind daher als Pharmaka zur systemischen Applikation bei Osteoporose bereits im klinischen Einsatz (Reginster, J.-Y.: Curr. Pharmaceut. Design 8 (2002), 1907-1916).

Es wurden in der Vergangenheit zahlreiche Anstrengungen unternommen, die Osteointegration verbessernde hoch- oder niedermolekulare Substanzen auf unbeschichteten sowie auf Calciumphosphat- oder andersartig beschichteten Implantatoberflächen zu immobilisieren. Da eine rein adsorptive Immobilisierung auf Implantatoberflächen, beispielsweise durch Tauchen oder Besprühen derselben mit geeigneten Lösungen der die Osteointegration verbessernden Substanzen den Nachteil einer schnellen Auswaschung der Substanzen aus der Oberfläche mit sich bringt, wurden unterschiedliche Verfahren einer dauerhaften Immobilisierung vorgeschlagen. Beispiele sind die kovalente Fixierung von Bisphosphonaten auf Calciumphosphat-Oberflächen (Peter B. et al., Local delivery of bisphosphonate from coated orthopedic implants increases implants mechanical stability in osteoporotic rats., J Biomed Mater Res A. 2006 Jan;76(1):133-43) oder die Beschichtung von metallischen Implantaten mit Suspensionen aus resorbierbaren Polymeren und BMP (Schmidmaier et al., Collective review: bioactive implants coated with poly(D,L-lactide) and growth factors IGF-I, TGF-betal, or BMP-2 for stimulation of fracture healing, J Long Term Eff Med Implants. 2006;16(1):61-9). Wesentliche Nachteile all dieser Verfahren sind jedoch darin zu sehen, dass die Aufbringung der die Osteointegration verbessernden Komponente mit einem hohen technischen Aufwand verbunden ist und zusätzliche Hilfsstoffe wie Polymere, organische Lösungsmittel oder Kopplungsagenzien benötigt werden, die das Einwachverhalten der Implantate negativ beeinflussen können. Im Falle einer wünschenswerten Immobilisierung von wasserlöslichen Strontiumsalzen auf Implantatoberflächen besteht darüber hinaus das Ziel einer stetigen kontrollierten Freisetzung geringer Dosen von Strontiumionen aus der Implantatoberfläche über einen Zeitraum von mehreren Tagen bis Wochen, da bekannt ist, dass eine Überdosierung mit Strontiumionen zu cytotoxischen Effekten führen kann. Implantatbeschichtungen, die solche Anforderungen erfüllen, sind gegenwärtig nicht bekannt.

Die EP 1 481 696 beschreibt ein Verfahren zur Herstellung eines Implantates mit einer bioaktiven, Strontium-substituierten keramischen Apatit-Beschichtung. Dazu wird das noch nicht beschichtete Implantat in eine Lösung aus Strontiumionen, Calciumionen und Phosphationen eingetaucht dann bei einem pH-Wert von 5 - 8, während einer Dauer von mehreren Stunden und einer Temperatur zwischen 30°C und 50°C inkubiert. Anschliessend wird das Implantat getrocknet. Es wird behauptet, dass diese Inkubation zu einer Apatit-Beschichtung führt, bei welcher Teile der Ca-Ionen substituiert sind durch Strontiumionen. Aussagen über die Haftungseigenschaften einer derartigen Schicht werden nicht gemacht. Es zeigt sich aber, dass derartige Apatit-Beschichtungen eine sehr ungenügende Haftung auf dem darunter liegenden Substrat, sei es nun metallisch oder Keramik, aufweisen.

Aus der WO 03/039609 ist ein Verfahren zur Beschichtung von Implantaten bekannt, bei welchem eine mehrere Lagen umfassende Schicht auf einem Titan-Implantat hergestellt wird, indem zunächst die Oberfläche in einem Säurebad elektrolytisch gereinigt wird, wobei auch eine Wechselspannung angelegt werden kann, und wobei die Oberflächenschicht entfernt wird. Anschliessend wird in einem Phosphorsäurebad gezielt eine Oxidschicht aufgebaut. In einem dritten Bad, in welchem nun Kalzium und Phosphationen enthalten sind, wird ein elektrisches Potenzial zur Bildung einer Hydroxyapatit-Schicht angelegt. Dabei wird erwähnt, dass der angelegte Gleichstrom erhöht werden kann, bis ein Schmelzen in der Oberfläche abläuft. Im letzten Schritt wird die oberste Schicht aufgebracht, indem das Implantat in eine Lösung mit Vesikeln, mit einer Innenschicht mit Phospholipid und einer Aussenschicht aus Kalziumsphosphat, eingetaucht wird, und unter Anlegen wiederum eines elektrischen Potenzials eine Ablagerung auf dem Implantat stattfindet. Dabei wird unter anderem darüber gesprochen, dass das in den Vesikeln enthaltene Kalzium im Kalziumsphosphat teilweise ersetzt werden kann durch Magnesium, Strontium, Barium oder eine Mischung dieser Ionen, dies beispielsweise, um die Haftung der Schicht zu verbessern. Eine plasmachemische Behandlung findet dabei nicht statt.

Auch aus der US 2005/0000819 ist ein Verfahren zur Oberflächenbeschichtung von Titan-Implantaten bekannt. Dabei wird ebenfalls Kalziumsphosphat elektrochemisch auf der Oberfläche abgeschieden. Das darin beschriebene Verfahren wird als MECD (Modulated electrochemical deposition) bezeichnet, und es beruht auf der gepulsten Anlegung einer Spannung, wobei Rechteckimpulse mit einer Länge von 200µs-20s eingesetzt werden. Die Modulationsfrequenz bewegt sich mit anderen Worten höchstens im Bereich von einigen Herz. Die Stromdichte beim verwendeten Verfahren liegt im Bereich von 200 µA/cm² bis 3 mA/cm². Damit liegt auch bei diesem Verfahren keine plasmachemische Oxidation vor.

Die WO2006104644 beschreibt ein oberflächenmodifiziertes Implantat mit einer Vielzahl von Nanoröhren und ein Verfahren zur Herstellung eines derart an der Oberfläche modifizierten Implantats. Die metall-haltige Oberfläche des Implantats wird dabei modifiziert mit Hilfe einer elektrochemischen Anodisierung, um eine Vielzahl von Nanoröhrchen aus einem Oxid des Metalls auf mindestens einer Oberfläche des Implantats auszubilden.

### DARSTELLUNG DER ERFINDUNG

Der Erfindung liegt die Aufgabe zugrunde, eine verbesserte bioaktive, auf einem Substrat insbesondere fest haftende Beschichtung sowie ein Verfahren zu deren Herstellung auf einem Substrat zur Verfügung zu stellen, die eine gute, komplikationslose Osteointegration zeigt und gleichzeitig in einem einfachen und kostengünstigen Verfahren hergestellt werden kann.

Erfindungsgemäss wird die Aufgabe gelöst, in dem eine auf der Implantatoberfläche festhaftende, poröse Beschichtung bereitgestellt wird, die als wirksamen Schichtbestandteil Strontiumionen in Form eines variablen Anteils einer in wässrigen Medien schwerlöslichen Strontiumverbindung enthält.

Insbesondere wird die Aufgabe dadurch gelöst, indem ein Verfahren vorgeschlagen wird zur Beschichtung eines Dentalimplantates auf metallischer Basis, welches
dadurch gekennzeichnet ist, dass das Implantat wenigstens in einem im implantierten Zustand dem Knochen ausgesetzten Bereich in eine wässrige (Elektrolyt-)Lösung mit Strontiumionen eingetaucht wird, und die Beschichtung in einer plasmachemischen anodischen Oxidation gebildet wird.

Es zeigt sich, dass bei der Verwendung eines derartigen Verfahrens zur Beschichtung einerseits das Strontium hervorragend in einer Oberflächenschicht gebunden wird und so verhindert werden kann, dass die Strontiumionen in die Umgebung in einer Art und Weise abgegeben werden, dass die Konzentration in der Umgebung zu hoch wird und damit nicht mehr ein das Wachstum fördernder Effekt sondern ein toxischer Effekt bewirkt wird. Des weiteren wurde gefunden, dass derart hergestellte Schichten eine hervorragende Haftfestigkeit auf dem Substrat zeigen. Solche Beschichtungen können entweder in einem einzigen Schritt oder in mehreren Arbeitsgängen erzeugt werden. Dabei können auch für die unterschiedlichen Arbeitsgänge unterschiedliche ElektrolytLösungen verwendet werden und entsprechend unterschiedliche Schichten erzeugt werden.

Bei der anodischen plasmachemischen Oxidation handelt es sich um ein an sich bekanntes Verfahren, welches aber noch nie im Zusammenhang mit der Einbringung von Strontiumionen in eine Beschichtung für Implantate Verwendung gefunden hat. Beschreibungen der plasmachemischen anodischen Oxidation finden sich beispielsweise für Kalziumsphosphatsbeschichtungen in der US 5 205 921 sowie generell für keramische Oxidschichten beispielsweise in der US 5 811 194, der US 5 385 662, der US 4 846 837, oder der US 5 478 237.

Diese anodische Oxidation, wie z.B. in der US 5 811 194 beschrieben, ist in wässrigen Elektrolyten eine Gas-Festkörper-Reaktion unter Plasmabedingungen, bei der der hohe Energieeintrag am Fusspunkt der Entladungssäule auf der Anode flüssiges Metall erzeugt, das mit dem aktivierten Sauerstoff ein kurzzeitverschmolzenes Oxid bildet. Die Schichtbildung erfolgt über Partialanoden. Der Funkenentladung ist ein Formierbereich vorgelagert. Die Elektrolyte werden so kombiniert, dass ihre positiven Eigenschaften vereint werden und qualitativ hochwertige anodisch erzeugte Oxidkeramikschichten auf Metallen entstehen. Durch Kombination verschiedener Salze können höhere Salzkonzentrationen im Elektrolytbad und damit höhere Viskositäten erreicht werden. Solche hochviskosen Elektrolyte haben eine hohe Wärmekapazität, stabilisieren den ausgebildeten Sauerstofffilm auf der Anode und garantieren damit eine gleichmässige Oxidschichtausbildung.

Auf dem Metall oder der Metalllegierung befindet sich natürlicherweise eine Sperrschicht. Durch Erhöhung der Spannung des anodisch gepolten Metalls wächst die Sperrschicht. Dann entsteht an der Phasengrenze Metall/Gas/Elektrolyt partiell ein Sauerstoffplasma, durch das sich die Oxidkeramikschicht bildet. Das Metallion in der Oxidkeramikschicht stammt aus dem Metall, der Sauerstoff aus der anodischen Reaktion in dem verwendeten wässrigen Elektrolyten. Die Oxidkeramik ist bei den ermittelten Plasmatemperaturen von etwa 7.000 Kelvin flüssig. Zur Seite des Metalls hin ist die Zeit ausreichend, damit sich die Schmelze der Oxidkeramik gut zusammenziehen kann und so eine porenarme Oxidkeramikschicht bildet. Zur Seite des Elektrolyten hin wird die Schmelze der Oxidkeramik schnell durch den Elektrolyten abgekühlt und die noch abwandernden Gase, insbesondere Sauerstoff und Wasserdampf hinterlassen eine Oxidkeramikschicht mit einem weitmaschig verknüpften Kapillarsystem.

Damit tatsächlich eine plasmachemische anodische Oxidation im Sinne der vorliegenden Erfindung abläuft, muss die Stromdichte über 5 mA/cm² sein, bevorzugt oberhalb von 10, insbesondere bevorzugt oberhalb von 50 mA/cm². Liegt die Stromdichte unterhalb von einem solchen Wert, findet eine konventionelle elektrochemische Abscheidung statt und es bildet sich kein Plasma im Rahmen dieser Abscheidung.

Damit des weiteren eine plasmachemische anodische Oxidation im Sinne der vorliegenden Erfindung vorhanden ist, wird die angelegte Spannung mit einer hohen Frequenz im Kilohertz Bereich von wenigstens 500 Hz moduliert.

Gemäss einer bevorzugten Ausführungsform handelt es sich beim Substrat für das Implantat um ein Substrat auf Titan-Basis.

Gemäss einer weiteren bevorzugten Ausführungsform enthält die Elektrolyt-Lösung nicht nur Strontiumionen sondern auch weitere Ionen, welche in Form von löslichen Salzen eingebracht werden, wie z.B. zusätzlich Phosphationen, dies insbesondere bevorzugt in einer Konzentration im Bereich von 0.03 - 0.2 mol/l.

Alternativ oder zusätzlich ist es möglich, dass die Elektrolyt-Lösung zusätzlich Calciumionen enthält, dies insbesondere bevorzugt in einer Konzentration im Bereich von 0.06 - 0.12 mol/l.

Es erweist sich dabei als vorteilhaft, wenn die Strontiumionen in der Lösung in einer Konzentration im Bereich von 0.01 - 0.03 mol/l, insbesondere bevorzugt im Bereich von 0.01 - 0.02 mol/l, vorgelegt werden, dies wenn es sich um eine im wesentlichen wässrige Elektrolyt-Lösung handelt.

Eine weitere bevorzugte Ausführungsform zeichnet sich dadurch aus, dass das Implantat anodisch kontaktiert wird, dass zusätzlich eine Edelstahl-Katode in das Bad eingeaucht wird, und mit einem Gleichstrom die Oxidation ausgelöst wird, wobei eine bevorzugtermassen im Laufe der Beschichtungszeit zunehmende Spannung im Bereich von 100-500V insbesondere bevorzugt von 200-300 V angelegt wird. Dabei ist es möglich, dass gepulster Gleichstrom mit einer Frequenz im Bereich von 0.5-2 kHz bei einem Tastverhältnis von 1: 2 bis 2:1 verwendet wird. Wie erwähnt liegt die Frequenz typischerweise bei wenigstens 500 Hz.

Gemäss einer weiteren bevorzugten Ausführungsform ist es möglich, das Implantat vor (oder auch nach) der Beschichtung an der Oberfläche strukturiert wird. Es kann sich dabei um eine Makrostrukturierung handeln wie sie beispielsweise unter Zuhilfenahme einer Strahlbehandlung, und/oder aber auch um eine Mikrostrukturierung beispielsweise durch eine chemische Behandlung in einem Säurebad oder in einer Salzschmelze.

Typischerweise wird das Implantat nach einem oder mehreren derartigen Beschichtungsvorgängen mit Wasser, insbesondere bevorzugt destilliertem Wasser gespült und anschliessend getrocknet.

Es handelt sich bei der Elektrolyt-Lösung um eine wässrige Lösung. Es ist aber auch möglich, ein anderes insbesondere bevorzugt polares Lösungsmittel zu verwenden, oder Mischungen eines von Lösungsmitteln. Mögliche weitere Lösungsmittel sind beispielsweise Acetonitril, DMSO.

Wie bereits erläutert verfügt die Beschichtung, wenn sie mit einem derartigen Verfahren hergestellt wird, über eine ausgezeichnete Haftfestigkeit. Entsprechend ist gemäss einer bevorzugten Ausführungsform das Verfahren dadurch gekennzeichnet, dass die resultierende Beschichtung auf dem Substrat eine Haftfestigkeit von wenigstens 3 MPa, insbesondere bevorzugt von wenigstens 8 MPa aufweist.

Typischerweise verfügt die so hergestellte Beschichtung über eine sehr geeignete poröse Struktur für den Vorgang des Einwachsens. Bevorzugtermassen verfügt die poröse Struktur über eine Porendichte von 10⁴ bis 10⁷ Poren/mm² und/oder über eine durchschnittliche Porengrösse zwischen 0,2 und 5 µm.

Bevorzugtermassen verfügt eine derartige Beschichtung über eine Dicke im Bereich von 0.5-50 µm.

Zudem betrifft die vorliegende Erfindung eine bioaktive, auf einem Substrat (insbesondere einem metallischen Substrat) haftende Beschichtung für medizinische Implantate und Dentalimplantate, welche dadurch gekennzeichnet ist, dass sie nach einem Verfahren, wie oben beschrieben wurde, herstellbar oder hergestellt ist.

Die vorliegende Erfindung betrifft eine bioaktive, auf einem Substrat fest haftende Beschichtung für medizinische Implantate und Dentalimplantate, bevorzugtermassen welche dadurch gekennzeichnet ist, dass die Beschichtung Strontiumionen in Form von in wässrigen Medien schwerlöslichen Strontiumverbindungen enthält, der Strontiumgehalt in der Beschichtung 1 bis 50 Atomprozente, bevorzugt 5 - 30 Atomprozente, insbesondere bevorzugt 5 - 15 Atomprozente beträgt, und die Beschichtung eine poröse Struktur, insbesondere bevorzugt mit einer Porendichte von 10⁴ bis 10⁷ Poren/mm² und bevorzugtermassen mit einer durchschnittlichen Porengrösse zwischen 0,2 und 5 µm besitzt. Die Strontiumionen können dabei wenigstens teilweise in Form eines Strontiumphosphates und/oder eines Strontiumtitanates wie z.B. einer Perowskitstruktur oder einer perowskitähnlichen Struktur mit eingelagerten Strontiumionen vorliegen. Diese Formen können in amorpher oder nanokristalliner Form vorliegen.

Alternativ oder zusätzlich ist es möglich, dass die Beschichtung Strontiumionen enthält, welche in einer Mischoxid-Struktur, insbesondere einer Mischoxid-Struktu.r enthaltend Titan und/oder Calcium, insbesondere bevorzugt in einer Perowskit-Struktur der Beschichtung eingebettet sind.

In einer weiteren bevorzugten Ausführungsform der Erfindung enthält die Beschichtung amorphe und/oder kristalline Calciumphosphat-Phasen.

Die Strontium respektive die Calciumionen in der wässrigen Lösung können dabei vorzugsweise mit einem Chelat-Bildner in Form eines Komplexes vorliegen, so beispielsweise als EDTA-Komplex. Vor der Behandlung in der plasmachemischen Oxidation kann das Material bereits an der Oberfläche strukturiert sein, beispielsweise infolge einer Säurebehandlung (beispielsweise in einem Salpetersäure/Flusssäure-Bad) oder infolge einer Strahlbehandlung oder einer Kombination von solchen Verfahren.

Die Beschichtung verfügt am Ende vorzugsweise über eine Dicke im Bereich von 2-10 µm. Die Beschichtung verfügt vorzugsweise über Poren in Form von Sacklöchern mit einem typischen mittleren Durchmesser im Bereich von 0.5-3 µm.

Typischerweise verfügt die Beschichtung über einen Anteil von 20 bis 95 Atomprozenten Metalloxid, bestehend aus einem oder mehreren Metallen oder Mischungen unterschiedlicher Metalloxide. Bei dem Metalloxid handelt es sich beispielsweise und bevorzugtermassen um Titandioxid und/oder Calciumoxid oder davon gebildete Mischformen wie beispielsweise Perowskite. Die Schicht kann auch im wesentlichen vollständig aus einem derartigen Perowskit mit eingelagerten Strontiumionen bestehen.

Weiterhin betrifft die vorliegende Erfindung ein Substrat, insbesondere Implantat mit einer Beschichtung wie sie oben beschrieben wurde, welches dadurch gekennzeichnet ist, dass das Substrat eines der Elemente Ti, Ta, Nb, Zr, Al, Ca, Mg, V oder deren Legierungen enthält. Das Substrat kann dabei vollständig oder teilweise mit der Beschichtung bedeckt sein.

Weiterhin betrifft die vorliegende Erfindung die Verwendung einer derartigen bioaktiven Beschichtung zur vollständigen oder teilweisen Ausrüstung von metallischen Oberflächen von Implantaten im Medizinal- und Dentalbereich, wobei die metallischen Oberflächen bevorzugt glatt, strukturiert und/oder porös sind.

Weitere bevorzugte Ausführungsformen sind in den abhängigen Ansprüchen beschrieben.

Die durch die Erfindung erreichten Vorteile sind zusammenfassend also im wesentlichen darin zu sehen, dass dank der erfindungsgemässen Beschichtung der Knochen schneller in die poröse Trägerstruktur einwachsen kann. Dabei erfolgt durch die in der Beschichtung in Form von in Wasser schwerlöslichen Strontiumverbindungen vorhandenen Strontiumionen eine Stimulierung knochenbildender Zellen (Osteoblasten), so dass eine verbesserte Integration des Implantats in das umliegende Gewebe gegeben ist. Da die Strontiumionen in Form einer schwerlöslichen Strontiumverbindung in die Beschichtung integriert sind, ist die Konzentration an gelösten, frei beweglichen Strontiumionen in der Umgebung der Implantat-Grenzfläche gering, so dass zwar ausreichend Strontiumionen zur Stimulierung des Knochenwachstums vorhanden sind, aber keine Gefahr einer Überdosierung und damit einer cytotoxischen Wirkung von Strontiumionen auf die das Implantat umgebenden Zellen besteht.

Die Beschichtung kann einen Anteil von 20 bis 95 Atomprozenten Metalloxid bestehend aus einem oder mehreren Metallen oder Mischungen unterschiedlicher Metalloxide aufweisen. Wie bereits erläutert, beträgt die Dicke der Beschichtung bevorzugtermassen 0,5 bis 50 µm, vorzugsweise 0,5 bis 10 µm. Eine derartige, dünne Beschichtung verfügt über gute Hafteigenschaften auf dem Substrat und ist relativ stabil gegenüber mechanischer, insbesondere Scherbeanspruchung.

Die erfindungsgemässe Beschichtung wird, wie bereits erläutert, vorzugsweise auf Substrate aufgebracht, die aus in der Medizinal- und Dentaltechnik üblichen Metallen oder Metalllegierungen bestehen, vorzugsweise solchen Metallen oder Metalllegierungen, die eines oder mehrere der Elemente Ti, Ta, Nb, Zr, Al, Ca, Mg, V oder deren Legierungen enthalten. Die Substrate können dabei eine beliebige Form und Oberflächenmorphologie besitzen und die Beschichtung kann das Substrat vollständig oder teilweise erfassen.

Die erfindungsgemässe Beschichtung wird mittels dem Verfahren der plasmachemischen anodischen Oxidation auf das zu beschichtende Implantat aufgebracht. Der in diesem Verfahren eingesetzte wässrige Elektrolyt enthält dabei Strontium-, bevorzugtermassen Phosphat- und gegebenenfalls weitere Ionen, welche Bestandteile der erfindungsgemässen Beschichtung sein sollen, in Form geeigneter wasserlöslicher Salze. Der für die plasmachemische Oxidation verwendete Elektrolyt kann darüber hinaus weitere, den Prozess der Beschichtung verbessernde Zusätze wie Komplex- oder Chelatbildner oder den pH-Wert und/oder die Elektrolytleitfähigkeit einstellende Zusätze enthalten. Die Beschichtung entsteht durch Reaktion zwischen dem metallischen Substrat und den Komponenten des Elektrolyten.

### KURZE ERLÄUTERUNG DER FIGUREN

Die Erfindung soll nachfolgend anhand von Ausführungsbeispielen im Zusammenhang mit den Zeichnungen näher erläutert werden. Es zeigen:
- Fig. 1: Proteinbildung von Schweineosteoblasten auf Strontium- (Sr-P) und Strontium/Calcium (Ca-Sr-P)-haltigen Oberflächen nach 7, 14 und 21 Tagen im Vergleich zu Calcium (Ca-P) -haltigen und nichtbeschichteten (Kontrolle) TiAl6V4-Oberflächen;
- Fig. 2: AP-Aktivität von Schweineosteoblasten auf Strontium (Sr-P)- und Strontium/Calcium (Ca-Sr-P)-haltigen Oberflächen nach 7, 14 und 21 Tagen im Vergleich zu Calcium (Ca-P)-haltigen und nichtbeschichteten (Kontrolle) TiA16V4-Öberflächen; und
- Fig. 3: SEM-Aufnahmen von unbeschichteten Titan-Oberflächen (al, Masseinheit 10 µm und a2, Masseinheit 2 µm) sowie von erfindungsgemäss beschichteten Titan-Oberflächen (b1, Masseinheit 10 µm; b2, Masseinheit 1 µm; c1, Masseinheit 10 µm; c2, Masseinheit 1 µm).

### WEGE ZUR AUSFÜHRUNG DER ERFINDUNG

Die nachstehenden Beispiele dienen der näheren Erläuterung der Erfindung. Die in der Folge diskutierten Ausführungsbeispiele sollen nicht dazu verwendet werden, den allgemein formulierten Gegenstand, wie er oben beschrieben ist sowie in den angehängten Ansprüchen formuliert ist, einschränkend auszulegen.

### Beispiel 1

Es wird ein wässriger Elektrolyt, in dem 20 g/l Strontiumacetat (C₄H₆O₄Sr*xH₂O) und 20 g/l Ethylendiaminotetraessigsäure (C₁₀H₁₆N₂O₈) sowie 10 g/l di-Natriumhydrogenphosphat 12-Hydrat (Na₂HPO₄.12H₂O) gelöst werden, hergestellt. In diesen wird eine Edelstahlkatode eingeführt und elektrisch mit einer äusseren Stromversorgungsquelle kontaktiert. Das zu beschichtende Substrat aus Titanium grade 2 wird anodisch kontaktiert und mit der Stromversorgung verbunden. Nach dem Einschalten der Stromversorgung wird bei einer konstanten Stromdichte von 5 A/dm² die Badspannung von 0 Volt beginnend, erhöht. Die Elektrolyttemperatur beträgt zwischen 15 und 40 °C. Es kommt gepulster Gleichstrom mit einer Frequenz von 1 kHz bei einem Tastverhältnis von 1:1 zur Anwendung. Bei Badspannungen von ca. 200 V zeigen sich erste plasmachemische Oxidationseffekte die anhand von visuell sichtbaren Funkenentladungen an der Anodenoberfläche verfolgt werden können. Wenn die Badspannung einen Wert von ca. 280 V erreicht hat, lässt man die Spannung nicht weiter steigen. Infolge dessen kommt es zu einem Absinken des Stromes. Wenn dieser die Hälfte des Ausgangswertes erreicht hat, wird der Beschichtungsprozess durch Ausschalten der Stromversorgung beendet. Das Titansubstrat wird aus dem Elektrolyt entfernt, mehrmals in destilliertem Wasser gespült und an Luft getrocknet. Die Titanoberfläche weist eine charakteristische mikroporöse Oberfläche bei Schichtdicken zwischen 5 und 10 µm auf. Die energiedispersive Röntgenanalyse der Schicht zeigt deutliche Gehalte an Sr, P, Ti und O.

### Beispiel 2

Ein wässriger Elektrolyt, in dem 50 g/l Ethylendiaminotetraessigsäure Calcium-di-Natriumsalz-Dihydrat, 20 g/l Ammoniumdihydrogenphosphat, 30 g/l Ethylendiamintetraessigsäure-Magnesium-di-Natriumsalz und 1 g/l Ethylendiamintetraessigsäure gelöst sind, wird mittels Ammoniaklösung auf einen pH-Wert von 8,6 eingestellt. Danach werden 20 g/l Strontiumacetat (C₄H₆O₄Sr*xH₂O) zugegeben und unter Einsatz eines Rührwerkes gelöst.

Danach wird ein Implantat aus der Titanlegierung TiAl6V4 in einer wässrigen Lösung, mit 175 ml/l HNO₃ (65%-ig) und 175 ml/l HF (40%-ig) 2 bis 10 s gebeizt und in dem oben beschriebenen wässrigen Elektrolyten anodisch kontaktiert. Die plasmachemische Oxidation dieses Implantates erfolgt bei einer gepulsten Spannung mit einer Frequenz von 1 kHz und einer Stromdichte von 2 A/dm² bei einer Elektrolyttemperatur von 20 bis 40°C. Mit der Endspannung von 280 V wird eine Schichtdicke von 5 bis 10 µm erzielt. Die energiedispersive Röntgenanalyse der Schicht zeigt deutliche Gehalte an Ca, Ti, P, O und Sr. Die so erzeugte Schicht wird durch die Legierungselemente der Titanlegierung dunkler gefärbt als die Schicht auf Reintitan.

### Beispiel 3

Jeweils 15 entsprechend den Beispielen 1 und 2 beschichtete quadratische Probekörper (Ti-Al6V4) von 10 x 10 x 1 mm (Kontrolle: unbeschichtet) wurden einzeln in die Kavitäten einer 24-Well-Zellkulturplatte überführt, 30 min mit 70% Ethanol desinfiziert und in der Folge je 30 min zweimal mit 1 ml sterilem PBS und einmal mit 1 ml Nährmedium (Alpha-Medium, Biochrom AG, Berlin, Deutschland, mit Zusatz von 10% fötalem Kälberserum, 50 U/mL Penicillin, 0,05 mg/mL Streptomycin, 2 mM Glutaminersatz, 0,1 µM Dexamethason, 10 mM β-Glycerophosphat) bei Raumtemperatur inkubiert.

Anschliessend wurden die Probekörper mit Schweineosteoblasten (5. Passage nach Primärkultur) besiedelt, in dem jeweils 1 ml einer Zellsuspension (Zelldichte ca. 100 000 Zellen/ml) von frisch trypsinierten Zellen einer Vorkultur zugesetzt wurden, woraus eine Zellbesiedlung von ca. 50 000 Zellen/cm² resultierte. Die Zellen wurden bei 37°C in einer 5% CO₂-Atmosphäre kultiviert, wobei das Nährmedium alle 2 Tage erneuert wurde. Nach 4 Tagen wurden die Probekörper in eine neue 24 well-Zellkulturplatte überführt.

Zur Bestimmung des Proteingehaltes und der alkalischen Phosphatase (AP)-Aktivität der Zellen wurden nach 7, 14 und 21 Tagen jeweils 4 Probekörper jeder Beschichtungsvariante sowie der Kontrolle entnommen, zweimal mit 1 ml PBS gespült und die Zellen auf den Probekörpern durch Zugabe von jeweils 0,1 ml CytoBusterTM Proteinextraktionsreagenz (Merck Biosciences, Bad Soden, Deutschland) lysiert und von aliquoten Teilen des Lysats der Proteingehalt und die AP-Aktivität bestimmt.

Die Proteinbestimmung wurde als kolorimetrischer Test mit der Mikro-BCA (Bicinchoninic acid)-Methode nach Vorschrift des Herstellers (Perbio Science Deutschland, Bonn, Deutschland) durchgeführt. Die Absorptionsmessung nach der Farbreaktion erfolgte in transparenten 96-well-Platten mit Hilfe eines Multiwellplatten-Photometers Genios Pro (Tecan Deutschland, Crailsheim, Deutschland) bei 565 nm und die Ermittlung der Proteinkonzentration an Hand einer mit Rinderserumalbumin erstellten Eichkurve.

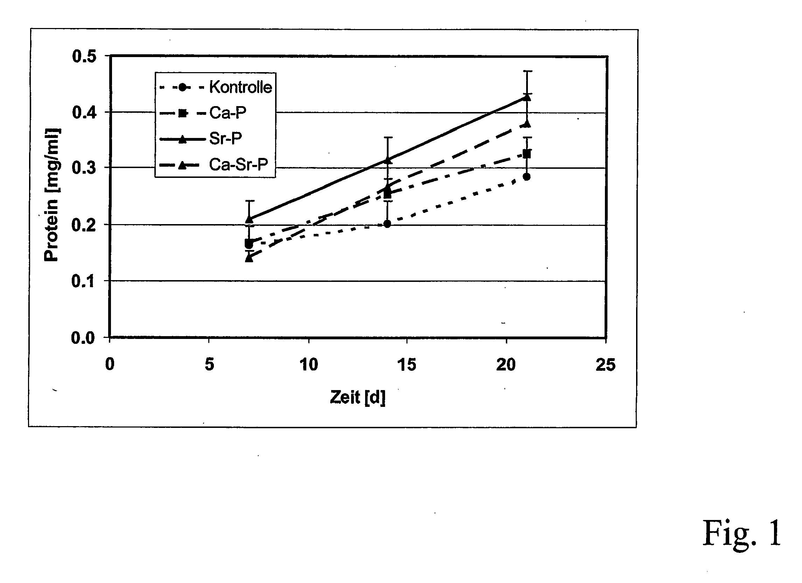

Fig. 1 zeigt die Proteinbildung von Schweineosteoblasten auf Strontium- und Strontium/Calcium-haltigen Oberflächen nach 7, 14 und 21 Tagen im Vergleich zu Calcium-haltigen und zu nichtbeschichteten TiAl6V4-Oberflächen.

Die AP-Aktivität wurde als Spaltungsaktivität gegenüber dem Lumineszenzsubstrat CDP-StarTM im alkalischen Milieu gemessen. Dazu wurden 5 µl Zelllysat mit 0,1 ml CDP-Star Ready-to-use Fertigreagenz mit Sapphire II-Enhancer (Applera Deutschland, Darmstadt, Deutschland) in schwarzen 96-well-Platten gemischt (2 Replikate je Zelllysat) und nach 20 min Inkubation bei Raumtemperatur die relative Lumineszenzintensität im Multiwellplatten-Photometer Genios Pro bei 28° C im Lumineszenzmodus gemessen. Die erhaltenen Lumineszenzwerte sind proportional zur AP-Aktivität, auf eine Bestimmung von Absolutwerten wurde verzichtet.

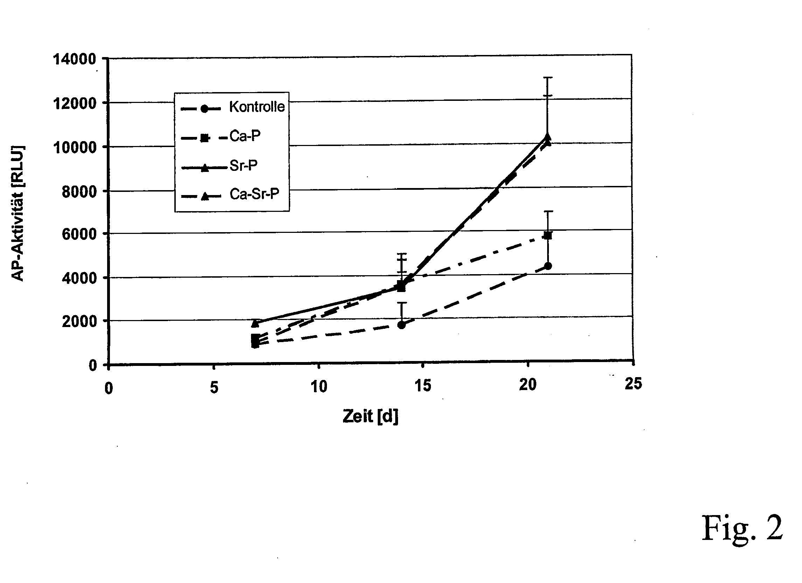

Fig. 2 zeigt die AP-Aktivität von Schweineosteoblasten auf Strontium- und Strontium/Calcium-haltigen Oberflächen nach 7, 14 und 21 Tagen im Vergleich zu Calcium-haltigen und zu nicht-beschichteten TiAl6V4-Oberflächen.

### Messung der Haftfestigkeit:

Zur Ermittlung der Haftfestigkeit der erfindungsgemässen Beschichtungen werden Titanprobekörper in den Abmessungen 20 x 10 x 2 mm (Länge x Breite x Höhe) nach Beispiel 1 beschichtet. Auf die beschichtete Oberfläche der Probekörper werden unter Verwendung eines kalthärtenden Epoxidharzklebstoffes (z. B. Epilox-System der Fa. Leuna Harze GmbH, Leuna, Deutschland) Edelstahlzylinder mit einem Durchmesser von 5 mm aufgeklebt, wobei die Klebefuge ca. 0,1 mm beträgt. In einer Instron-Zugprüfinaschine wurde anschliessend die Klebefestigkeit der aufgeklebten Zylinder ermittelt. Für eine Messung kamen 10 Probekörper zum Einsatz. Unter den gewählten Verklebungsbedingungen erfolgt der Schichtabriss ausschliesslich vom Untergrund der beschichteten Probekörper, so dass die ermittelten Werte die Haftfestigkeit der Beschichtung widerspiegeln.

Es wurde ein Wert für die Haftfestigkeit der Beschichtung von 9,9 MPa ermittelt.

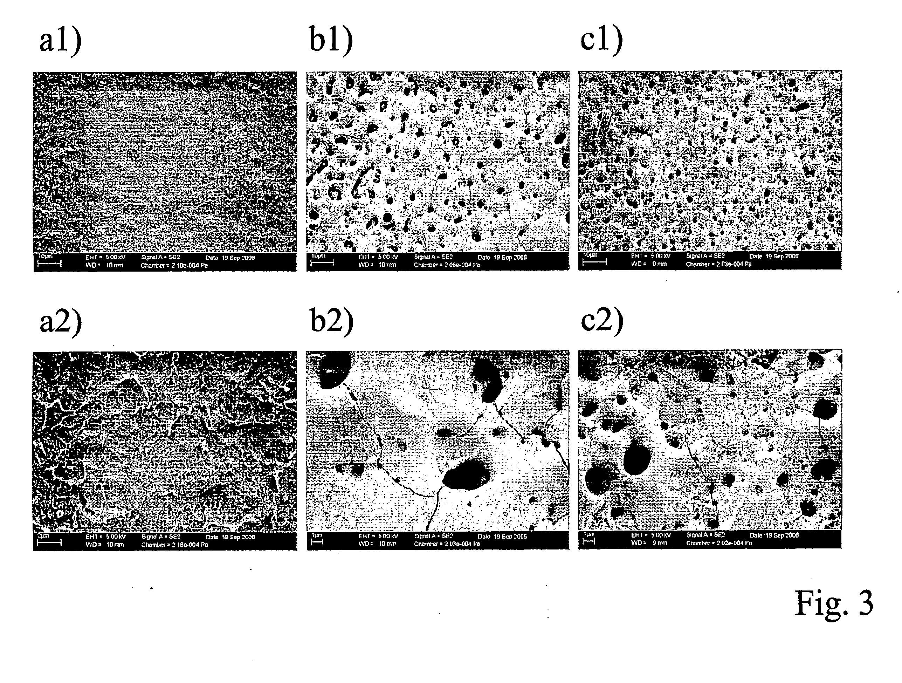

In Figur 3 sind SEM-Aufnahmen der Oberflächen dargestellt. In den Figuren a1 sowie a2 sind in unterschiedlichen Vergrösserungen die Oberflächen von unbehandelten TiAl6V4-Oberflächen gezeigt. Es zeigt sich, dass die Oberfläche im wesentlichen frei von Poren ist.

In den Figuren b1 und b2 sind in unterschiedlichen Vergrösserungen die Oberflächen von mit einer erfindungsgemässen Beschichtungen (Sr-P) versehenen TiAl6V4 Implantaten dargestellt (Verfahren nach Beispiel 1). Es zeigt sich, wie eine spezielle Art der Porenstruktur ausgebildet wird, die sich grundsätzlich von einer Oberflächen-Struktur unterscheidet, wie sie bei einer klassischen elektrochemischen Abscheidung entsteht, und wie sie beispielsweise in der US 2005/0000819 dargestellt ist, und wo das bei einer elektrochemischen Abscheidung erfolgende Kristallwachstum an der Oberflächenstruktur erkennbar bleibt.

In den Figuren c1 und c2 sind in unterschiedlichen Vergrösserungen die Oberflächen von mit einer erfindungsgemässen Beschichtung (Ca-Sr-P) versehenen TiAl6V4 Implantaten dargestellt. Auch hier findet man die ganz besondere Oberflächenstruktur, welche sich von einer Oberfläche aus einer klassischen elektrochemischen Abscheidung wesentlich unterscheidet.

## Patentansprüche

1. Verfahren zur Beschichtung eines medizinischen Dentalimplantates auf metallischer Basis,
**dadurch gekennzeichnet, dass**
das Implantat wenigstens in einem im implantierten Zustand dem Knochen ausgesetzten Bereich in eine wässrige Lösung mit Strontiumionen eingetaucht wird, und die Beschichtung in einer plasmachemischen anodischen Oxidation gebildet wird, wobei die Stromdichte über 5 mA/cm² liegt und die angelegte Spannung mit einer Frequenz von wenigstens 500 Hz moduliert wird.

2. Verfahren nach Anspruch 1, **dadurch gekennzeichnet, dass** es sich um ein Implantat auf Titan-Basis handelt.

3. Verfahren nach einem der vorhergehenden Ansprüche, **dadurch gekennzeichnet, dass** die Lösung zusätzlich Phosphationen enthält, insbesondere bevorzugt in einer Konzentration im Bereich von 0.03 - 0.2 mol/l.

4. Verfahren nach einem der vorhergehenden Ansprüche, **dadurch gekennzeichnet, dass** die Lösung zusätzlich Calciumionen enthält, insbesondere bevorzugt in einer Konzentration im Bereich von 0.06 - 0.12 mol/l.

5. Verfahren nach einem der vorhergehenden Ansprüche, **dadurch gekennzeichnet, dass** die Strontiumionen in der Lösung in einer Konzentration im Bereich von 0.01 - 0.03 mol/l, insbesondere bevorzugt im Bereich von 0.01 - 0.02 mol/l, vorgelegt werden.

6. Verfahren nach einem der vorhergehenden Ansprüche, **dadurch gekennzeichnet, dass** das Implantat anodisch kontaktiert wird, dass zusätzlich eine Edelstahl-Katode in das Bad eingetaucht wird, und mit einem Gleichstrom die Oxidation ausgelöst wird, wobei eine bevorzugtermassen im Laufe der Beschichtungszeit zunehmende Spannung im Bereich von 100-500V insbesondere bevorzugt von 200-300 V angelegt wird.

7. Verfahren nach Anspruch 6, **dadurch gekennzeichnet, dass** gepulster Gleichstrom mit einer Frequenz im Bereich von 0.5-2 kHz bei einem Tastverhältnis von 1: 2 bis 2:1 verwendet wird.

8. Verfahren nach einem der vorhergehenden Ansprüche, **dadurch gekennzeichnet, dass** die Stromdichte oberhalb von 10, insbesondere bevorzugt oberhalb von 50 mA/cm² liegt.

9. Verfahren nach einem der vorhergehenden Ansprüche, **dadurch gekennzeichnet, dass** das Implantat vor der Beschichtung an der Oberfläche strukturiert wird, insbesondere bevorzugt durch eine Strahlbehandlung.

10. Verfahren nach einem der vorhergehenden Ansprüche, **dadurch gekennzeichnet, dass** das Implantat nach einem oder mehreren Beschichtungsvorgängen mit Wasser, insbesondere bevorzugt destilliertem Wasser gespült und anschliessend getrocknet wird, und/oder dass die Beschichtung auf dem Substrat eine Haftfestigkeit von wenigstens 3 MPa, insbesondere bevorzugt von wenigstens 8 MPa aufweist, und/oder dass die Beschichtung eine poröse Struktur mit einer Porendichte von 10⁴ bis 10⁷ Poren/mm² und/oder einer durchschnittliche Porengrösse zwischen 0,2 und 5 µm und/oder eine Dicke im Bereich von 0.5-50 µm aufweist.

11. Bioaktive, auf einem Substrat haftende Beschichtung, **dadurch gekennzeichnet, dass** sie nach einem Verfahren nach einem der vorhergehenden Ansprüche herstellbar oder hergestellt ist.

12. Bioaktive, auf einem Substrat fest haftende Beschichtung nach Anspruch 11 für medizinische Implantate und Dentalimplantate, **dadurch gekennzeichnet, dass** die Beschichtung Strontiumionen in Form von in wässrigen Medien schwerlöslichen Strontiumverbindungen enthält, der Strontiumgehalt in der Beschichtung 1 bis 50 Atomprozente, insbesondere bevorzugt 5 - 15 Atomprozente beträgt, und die Beschichtung eine poröse Struktur mit einer Porendichte von 10⁴ bis 10⁷ Poren/mm² und einer durchschnittliche Porengrösse zwischen 0,2 und 5 µm besitzt wobei vorzugsweise Strontiumionen in Form eines Strontiumphosphates, bevorzugt in amorpher oder nanokristalliner Form, vorliegen und/oder wobei die Beschichtung eine Dicke im Bereich von 0.5-50 µm aufweist.

13. Beschichtung nach einem der Ansprüche 11-12, **dadurch gekennzeichnet, dass** sie Strontiumionen enthält, welche in einer Mischoxid-Struktur, insbesondere einer Mischoxid-Struktur enthaltend Titan und/oder Calcium, insbesondere bevorzugt in einer Perowskit-Struktur der Beschichtung eingebettet sind, und/oder dass die Beschichtung einen Anteil von 20 bis 95 Atomprozenten Metalloxid, bestehend aus einem oder mehreren Metallen oder Mischungen unterschiedlicher Metalloxide, enthält, wobei es sich bei dem Metalloxid vorzugsweise um Titandioxid handelt.

14. Substrat in Form eines Implantats mit einer Beschichtung gemäss einem der Ansprüche 11-13, **dadurch gekennzeichnet, dass** das Substrat eines der Elemente Ti, Ta, Nb, Zr, Al, Ca, Mg, V oder deren Legierungen enthält, wobei das Substrat vorzugsweise vollständig oder partiell mit der Beschichtung bedeckt ist.

15. Verwendung der bioaktiven Beschichtung nach einem der Ansprüche 11-13 zur vollständigen oder teilweisen Ausrüstung von metallischen Oberflächen von Implantaten im Medizinal- und Dentalbereich, wobei die metallischen Oberflächen bevorzugt glatt, strukturiert und/oder porös sind.

## Claims

1. A method for coating a metal-based dental implant, **characterized in that**
the implant is dipped into an aqueous electrolyte solution comprising strontium ions at least in an area exposed to the bone in the implanted state, and the coating is formed in a plasma-chemical anodic oxidation, wherein the current density is above 5 mA/cm² and the applied voltage is modulated with a frequency of at least 500 Hz.

2. A method according to claim 1, **characterized in that** it relates to a titanium-based implant.

3. A method according to any one of the preceding claims, **characterized in that** the solution additionally contains phosphate ions, preferably in a concentration in the range of 0.03-0.2 mol/l.

4. A method according to any one of the preceding claims, **characterized in that** the solution additionally contains calcium ions, preferably in a concentration in the range of 0.06-0.12 mol/l.

5. A method according to any one of the preceding claims, **characterized in that** the strontium ions in the solution are present in a concentration in the range of 0.01-0.03 mol/l, preferably in the range of 0.01-0.02 mol/l.

6. A method according to any one of the preceding claims, **characterized in that** the implant is anodically contacted, **in that** additionally a stainless steel cathode is dipped in the bath and the oxidation is triggered with a direct current, wherein a voltage in the range of 100-500 V, preferably increasing in the course of the coating time, preferably of 200-300 V, is applied.

7. A method according to claim 6, **characterized in that** pulsed direct current is used with a frequency in the range of 0.5-2 kHz at a pulse ratio of 1:2 to 2:1.

8. A method according to any one of the preceding claims, **characterized in that** the current density is above 10, preferably above 50 mA/cm².

9. A method according to any one of the preceding claims, **characterized in that** before the coating, the implant is structured on the surface, preferably through a blasting treatment.

10. A method according to any one of the preceding claims, **characterized in that** after one or several coating processes the implant is rinsed with water, preferably with distilled water, and subsequently dried, and/or that the coating on the substrate has an adhesive strength of at least 3 MPa, preferably of at least 8 MPa, and/or that the coating has a porous structure with a pore density of 10⁴ to 10⁷ pores/mm² and/or an average pore size between 0.2 and 5 µm and/or a thickness in the range of 0.5-50 µm.

11. A bioactive coating, adhering on a substrate, **characterized in that** it is producible or produced according to a method according to one of the preceding claims.

12. A bioactive coating firmly adhering on a substrate according to claim 11 for medical implants and dental implants, **characterized in that** the coating contains strontium ions in form of strontium compounds poorly soluble in aqueous media, the strontium content in the coating is 1 to 50 atomic percent, preferably 5-15 atomic percent, and the coating has a porous structure with a pore density of 10⁴ to 10⁷ pores/mm² and an average pore size between 0.2 and 5 µm, wherein there are preferably strontium ions in form of a strontium phosphate, preferably in amorphous or nano-crystalline form, and/or wherein the coating has a thickness in the range of 0.5-50 µm.

13. A coating according to one of the claims 11-12, **characterized in that** it contains strontium ions which are embedded in a mixed oxide structure, in particular a mixed oxide structure containing titanium and/or calcium, preferably in a Perovskite structure of the coating, and/or that the coating comprises a fraction of 20 to 95 atomic percent of metal oxide, consisting of one or several metals or mixtures of different metal oxides, wherein the metal oxide is preferably titanium dioxide.

14. A substrate in form of an implant with a coating according to one of the claims 11-13, **characterized in that** the substrate contains one of the elements Ti, Ta, Nb, Zr, Al, Ca, Mg, V or alloys thereof, wherein the substrate is preferably completely or partially covered with the coating.

15. Use of the bioactive coating according to one of the claims 11-13 for the complete or partial equipment of metallic surfaces of implants in the medical and dental field, wherein the metallic surfaces are preferably smooth, structured, and/or porous.

## Revendications

1. Procédé de revêtement d'un implant à usage médical et dentaire à base métallique,
**caractérisé en ce que**
l'implant, au moins dans une région exposée à l'os une fois implanté, est plongé dans une solution aqueuse contenant des ions strontium, et le revêtement est formé par oxydation anodique par voie plasma-chimique, la densité de courant étant supérieure à 5 mA/cm² et la tension appliquée étant modulée à une fréquence d'au moins 500 Hz.

2. Procédé selon la revendication 1, **caractérisé en ce qu'**il s'agit d'un implant à base de titane.

3. Procédé selon l'une des revendications précédentes, **caractérisé en ce que** la solution contient en plus des ions phosphates, de préférence à une concentration de l'ordre de 0,03 à 0,2 mole/l.

4. Procédé selon l'une des revendications précédentes, **caractérisé en ce que** la solution contient en plus des ions calcium, de préférence à une concentration de l'ordre de 0,6 à 0,12 mole/l.

5. Procédé selon l'une des revendications précédentes, **caractérisé en ce que** les ions strontium dans la solution sont à une concentration de l'ordre de 0,01 à 0,03 mole/l, de préférence de l'ordre de 0,01 à 0,02 mole/l.

6. Procédé selon l'une des revendications précédentes, **caractérisé en ce que** le contact avec l'implant est anodique, **en ce qu'**une cathode en acier fin est en plus plongée dans le bain, et l'oxydation est déclenchée par un courant continu, une tension de préférence croissante au cours du revêtement de l'ordre de 100 à 500 V, de préférence de 200 à 300 V, étant appliquée.

7. Procédé selon la revendication 6, **caractérisé en ce que** l'on utilise un courant continu pulsé d'une fréquence de l'ordre de 0,5 à 2 kHz dans un rapport cyclique de 1:2 à 2:1.

8. Procédé selon l'une des revendications précédentes, **caractérisé en ce que** la densité de courant est supérieure à 10, de préférence supérieure à 50 mA/cm².

9. Procédé selon l'une des revendications précédentes, **caractérisé en ce que** l'implant est doté de structures sur la surface avant le revêtement, de préférence par un traitement par irradiation.

10. Procédé selon l'une des revendications précédentes, **caractérisé en ce que** après un ou plusieurs processus de revêtement l'implant est aspergé d'eau, de préférence d'eau distillée, puis séché, et/ou **en ce que** le revêtement sur le substrat présente une adhérence d'au moins 3 MPa, de préférence d'au moins 8 MPa, et/ou **en ce que** le revêtement présente une structure poreuse ayant une densité de pores de 10⁴ à 10⁷ pores /mm² et/ou une dimension de pore moyenne comprise entre 0,2 et 5 µm et/ou une épaisseur de l'ordre de 0,5 à 50 µm.

11. Revêtement bioactif adhérant sur un substrat, **caractérisé en ce qu'**il est réalisé ou peut être réalisé conformément à un procédé selon l'une des revendications précédentes.

12. Revêtement bioactif selon la revendication 11, adhérant solidement sur un substrat et destiné à des implants à usage médical et des implants dentaires, **caractérisé en ce que** le revêtement contient des ions strontium sous la forme de composés du strontium difficilement solubles en milieux aqueux, la teneur en strontium dans le revêtement étant en pourcentage atomique de 1 à 50%, de préférence de 5 à 15%, et le revêtement présente une structure poreuse ayant une densité de pores de 10⁴ à 10⁷ pores/mm² et une dimension de pore moyenne comprise entre 0,2 et 5 µm, les ions strontium se présentant de préférence sous la forme d'un phosphate de strontium, de préférence sous une forme amorphe ou nanocristalline, et/ou le revêtement ayant une épaisseur de l'ordre de 0,5 à 50 µm.

13. Revêtement selon l'une des revendications 11-12, **caractérisé en ce qu'**il contient des ions strontium qui sont incorporés dans une structure d'oxyde mixte, notamment une structure d'oxyde mixte contenant du titane et/ou du calcium, de préférence dans une structure perovskitique du revêtement, et/ou **en ce que** le revêtement contient un pourcentage atomique d'oxydes métalliques de 20 à 95%, constitué d'un ou plusieurs métaux ou mélanges de différents oxydes métalliques, l'oxyde métallique étant de préférence du dioxyde de titane.

14. Substrat se présentant sous la forme d'un implant doté d'un revêtement selon l'une des revendications 11-13, **caractérisé en ce que** le substrat contient un des éléments Ti, Ta, Nb, Zr, Al, Ca, Mg, V ou leurs alliages, le substrat étant recouvert partiellement ou de préférence totalement avec le revêtement.

15. Utilisation du revêtement bioactif selon l'une des revendications 11-13 pour doter totalement ou partiellement des implants à usage médical et dentaire de surfaces métalliques, les surfaces métalliques étant de préférence lisses, structurées et/ou poreuses.
